# EUROPEAN PATENT SPECIFICATION

(11) **EP 1 924 235 B1**
(45) Date of publication and mention of the grant of the patent: **12.01.2011**
(21) Application number: 06813597.9
(22) Date of filing: 18.08.2006
(51) Int. Cl.: A61K 6/00, A61K 6/083

(54) **METHOD FOR BONDING ARTIFICIAL PLASTIC TEETH**
VERFAHREN ZUR BINDUNG VON KÜNSTLICHEN KUNSTSTOFFZÄHNEN
PROCEDE DE COLLAGE DE DENTS ARTIFICIELLES EN PLASTIQUE

(30) Priority: 18.08.2005 US 709152 P
(43) Date of publication of application: 28.05.2008
(73) Proprietor: DENTSPLY International Inc., York, PA 17405-0872 (US)
(72) Inventor: WEBER, Frederick, J., Thomasville, PA 17364 (US); SUN, Benjamin, Jieman, York, PA 17402 (US); GHERGULESCU, Camilia Maria, York, PA 17402 (US); YOUNG, Andrew, Mathias, Dallastown, PA 17313 (US); DEEBEL, Michelle, Mount Wolf, PA 17347 (US)
(74) Representative: Blodig, Wolfgang
(86) International application number: PCT/US2006/032580
(87) International publication number: WO 2007/022503

(56) References cited:
- EP-A1- 1 348 416
- EP-A2- 0 142 172
- EP-A2- 0 142 172
- JP-A- 7 291 816
- JP-A- 63 017 815
- RUPP, N.W.; BOWEN, R.L.; PAFFENBARGER, G.C.: "Bonding cold-curing denture base acrylic resin to acrylic resin teeth" JOURNAL OF THE AMERICAN DENTAL ASSOCIATION, vol. 83, September 1971 (1971-09), pages 601-606, XPxp008115703 ISSN: 0002-8177
- RUPP, N.W.; BOWEN, R.L.; PAFFENBARGER, G.C.: "Bonding cold-curing denture base acrylic resin to acrylic resin teeth" JOURNAL OF THE AMERICAN DENTAL ASSOCIATION, vol. 83, September 1971 (1971-09), pages 601-606, XPxp008115703 ISSN: 0002-8177

## Description

### Technical Field

The present invention is directed toward a method for bonding artificial plastic teeth to a denture base. The method according to the invention includes exposing a plastic tooth to an adhesive and allowing the plastic to soften (not dissolve). The adhesion promoter chains infiltrate the plastic tooth matrix, such that upon curing a secure bond is formed.

### Background of the Invention

The durability and longevity of the bond between synthetic polymer denture teeth and acrylic denture base resins has been a source of concern to both Dentists and Dental Technicians for many years. While a number of successful adhesives have been commercialized, shortcomings have been identified in these products, including for example, they are generally not suitable for use with more than pour and pack type acrylic denture bases, and the shelf-stability of the products has been limited by the presence of residual benzoyl peroxide initiator in the polymer component of the formulation. This leads to the eventual gelation of many of the known formulations.

A need exists therefore, for an improved bonding agent that addresses the previous liabilities. The adhesive or bonding agent should be particularly effective in initiating and maintaining the bond between acrylic denture teeth and both pour and light-curable denture base resins.

### Summary of the Invention

The present invention is directed toward a method for bonding artificial plastic teeth to a denture base. The adhesive used in this method employs a number of adhesion promoter monomers, dimers or oligomers and a cure package, and a solvent such as methyl acetate. The method according to the invention includes exposing a plastic tooth to the adhesive while warming the tooth and allowing the plastic to soften (not dissolve). The adhesion promoter chains infiltrate the plastic tooth matrix, such that upon curing a secure bond is formed.

### Brief Description of the Drawings

Figure 1 depicts fracture surfaces of bond test specimens prepared according to the present invention.

Figure 2 depicts fracture surfaces of bond test specimens prepared according to the present invention.

### Preferred Embodiments for Carrying Out the Invention

The chemistry of the bonding agent is formulated so as to enable reactive dimers and oligomers to diffuse into the surface structure of the teeth. These reactive entities, once initiated, undergo addition type polymerization across the interface between the teeth and the denture base resin to yield a strong and long-lived bond. The solvent works on the surface of artificial teeth so that polymerizable components of the composition can penetrate into the surfaces of teeth. For example, the polymerizable components in the composition can penetrate into the surface of teeth with the help of the methyl acetate solvent. The polymerizable components of the composition will later polymerize to form interpenetrating polymer networks with subsequently applied acrylics and polymerizable resins.

The present adhesives have particular application to use in the Dental Laboratory, for the purpose of facilitating a long-lived bond between plastic denture teeth and addition cured denture base resins. Toward that end, the laboratory technician will prepare the teeth as necessary and will soak the ridgelap portion of the teeth in the inventive bonding agent in a closed container for a period of approximately 5-minutes while warming the tooth. The teeth will be removed from the container and be allowed to bench set for a period of 1-minute. During this period, in a manner similar to that of the Trubyte Denture Bond available from DENTSPLY International of York, PA, the reactive components of the bonding agent will solidify on the teeth. The teeth may then be set into hydrocolloid investment for further processing as a pour acrylic case, or directly into uncured light-cure denture base resin for subsequent try-in and processing as in the manner of an Eclipse® denture (Eclipse is a trademark of DENTSPLY International).

The diffusion of the bonding agent components into the tooth structure has been found to be accelerated by warming the teeth while they are in contact with the inventive bonding agent formulation. This can be accomplished through the use of any one of a number of warming methods, but most preferably is accomplished through the use of a stand-alone electrical warming device onto which the tins, containing the teeth and the inventive bonding agent formulation, can be placed.

The present bonding agent may be supplied in a kit-type format with all the components necessary for treating denture teeth. The kit may also include not only the bonding agent itself but also a tin or other container used to treat the teeth, and the inserts for the tin which are die-cut pieces of superabsorbent foam that are swollen by the inventive bonding agent on contact and serve as positioners for the teeth while they are being treated.

### Polymerizable Compounds

One class of suitable polymerizable compounds that can be used in the composition contains materials having free radically active functional groups and includes monomers, oligomers, and polymers having one or more ethylenically unsaturated groups. Such free radically polymerizable compounds include, but are not limited to, mono-, di- or poly-acrylates and methacrylates such as methyl acrylate, methyl methacrylate, ethyl acrylate, isopropyl methacrylate, n-hexyl acrylate, stearyl acrylate, allyl acrylate, glycerol diacrylate, glycerol triacrylate, ethyleneglycol diacrylate, diethyleneglycol diacrylate, triethyleneglycol dimethacrylate, tetraethylene glycol di(meth)acrylate, 1,3-propanediol diacrylate, 1,3-propanediol dimethacrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, pentaerythritol triacrylate, pentaerythritol tetraacrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)phenyl]propane (Bis-GMA); 2,2-bis[4-(acryloyloxyethoxy)phenyl]propane; 2,2-bis[4-(methacryloyloxy-ethoxy)phenyl]propane (or ethoxylated bisphenol A-dimethacrylate) (EBPADMA); urethane di(meth)acrylate (UDMA), diurethane dimethacrylate (DUDMA), 4,13-dioxo-3,14 dioxa-5,12-diazahexadecane-1,16-diol diacrylate, 4,13-dioxo-3,14 dioxa-5,12-diazahexadecane-1,16-diol dimethacrylate; polyurethane dimethacrylate (PUDMA); alkoxylated pentacrythritol tetraacrylatel; polycarbonate dimethacrylate (PCDMA); the bis-acrylates and bis-methacrylates of polyethylene glycols; copolymerizable mixtures of acrylated monomers; acrylated oligomers; acidic monomers such as dipentaerythritol pentacrylate phosphoric acid ester (PENTA); bis[2-(methacryloxyloxy)-ethyl]phosphate; and vinyl compounds such as styrene, diallyl phthalate, divinyl succinate, divinyl adipate and divinylphthalate. The polymerizable compound can be used alone in the composition or mixtures of the polymerizable compounds can be used.

### Solvents

The composition of this invention includes a solvent that is capable of at least partially solubilizing the wax material. Examples of suitable solvents include, but are not limited to, acetone, ethyl acetate, propyl acetate, ethers, methylene chloride, chloroform, cyclohexanone, methyl acetate, methyl ethyl ketone, methyl propyl ketone, and tetrahydrofuran, etc. Preferably, methyl acetate is used as the solvent. The methyl acetate is able to penetrate into the cured baseplate material as described.

### Polymerization initiators

Polymerization initiators, such as peroxides, can be added to the composition to make it heat curable. The peroxides generate free radicals to initiate polymerization and hardening of the composition. Peroxides such as dibenzoyl peroxide (BPO), di-p-chlorobenzoyl peroxide, di-2,4-dichlorobenzoyl peroxide, tertiary butyl peroxybenzoate, methyl ethyl ketone peroxide, ditertiary butyl peroxide, dicumyl peroxide and cumene hydroperoxide, and the like can be added to the bonding agent.

### Polymerization accelerators

The bonding agent may further include a polymerization accelerator, which is preferably a tertiary amine. Examples of tertiary amines, which can be used in the bonding agent include, N, N-dimethyl-aminoneopentyl acrylate, N, N-dimethyl-aminoethyl acrylate, N, N-dimethyl-aminoethyl methacrylate, N-methyl-diethanolamine; ethyl 4-(dimethylamino)benzoate (EDMAB); 2-[4-(dimethylamino)phenyl] ethanol; N, N-dimethyl-p-toluidine (DMPT); dihydroxyethyl-p-toluidine (DHEPT); bis(hydroxyethyl)-p-toluidine; triethanolamine; and the like.

### Photoactive Agents

A photoactive agent such as, for example, benzophenone, benzoin and their derivatives, or alpha-diketones and their derivatives can be added to the bonding agent. A preferred photopolymerization initiator is camphorquinone (CQ). Photopolymerization can be initiated by irradiating the composition with blue, visible light preferably having a wavelength in the range of about 400 to about 500 nm. A standard dental blue light-curing unit can be used to irradiate the composition. The camphorquinone (CQ) compounds have a light absorbency maximum of between about 400 to about 500 nm and generate free radicals for polymerization when irradiated with light having a wavelength in this range. Alternatively, the photoinitiator can be selected from the class of acylphosphine oxides such as monoacyl phosphine oxide derivatives, bisacyl phosphine oxide derivatives, and triacyl phosphine oxide derivatives. For, example, 2,4,6-trimethylbenzoyl-diphenyl-phosphine oxide (TPO) can be used as the photopolymerization initiator. In another instance, a material referred to as "ALF" comprising camphorquinone (CQ); butylated hydroxytoluene (BHT); N,N-dimethylaminoneopentyl acrylate, and methacrylic acid can be used in the composition.

### Polymerization Inhibitors

In addition, the bonding agent may include a polymerization inhibitor such as, for example, butylated hydroxytoluene (BHT); hydroquinone; hydroquinone monomethyl ether; benzoquinone; chloranil; phenol; butyl hydroxyanaline (BHT); tertiary butyl hydroquinone (TBHQ); tocopherol (Vitamin E); and the like. Preferably, butylated hydroxytoluene (BHT) is used as the polymerization inhibitor. The polymerization inhibitors act as scavengers to trap free radicals in the resulting composition and to extend the working and setting time of the composition.

### Fillers

Conventional filler materials such as for example, inorganic fillers, which can be naturally-occurring or synthetic, can be added. Such materials include, but are not limited to, silica, titanium dioxide, iron oxides, silicon nitrides, glasses such as calcium, lead, lithium, cerium, tin, zirconium, strontium, barium, and aluminum-based glasses, borosilicate glasses, strontium borosilicate, barium silicate, lithium silicate, lithium alumina silicate, kaolin, quartz, and talc. Preferably, the silica is in the form of silanized fumed silica. Preferred glass fillers are silanized barium boron aluminosilicate and silanized fluoride barium boron aluminosilicate. Organic particles such as poly(methyl methacrylate), poly(methyl/ethyl methacrylate), crosslinked polyacrylates, polyurethanes, polyethylene, polypropylene, polycarbonates and polyepoxides , etc. also can be used as fillers..

The bonding agent composition of this invention contains semi-crystalline components. When the solvent in the composition evaporates, it may form a solid layer which acts as a bonding agent to adhere subsequently applied acrylics and other resins to the teeth's surfaces. The dried composition of this invention forms a hard, non-sticky surface layer that provides an excellent interface for subsequently applied acrylics and other resins to effectively bond to the surfaces of the artificial denture teeth. In contrast, compositions that form a liquid or non-solid surface layer feel sticky or tacky. With such a tacky interface, it is not easy to handle with fingers. At uncured stage, the bond created with a liquid or non-solid surface layer between the teeth and subsequently applied Eclipse® resins is not strong and the Eclipse® resins can delaminate from denture teeth prior to cure.

Components having low tackiness at a temperature in the range of room temperature to 37°C are preferably included in the composition of this invention. These components provide rapid solidification of the polymerizable products upon solvent evaporation. Polymerizable dental compositions of this invention also preferably contain rapidly partially recrystallizable components. Rapid recrystallizable components provide rapid solidification of the polymerizable products and a combination of flowability and dimensional stability depending upon their applied temperature and the solvents used. When polymerized, the crystallized phase melts effectively resulting in volume expansion, which offsets polymerization shrinkage. Thus, the polymeric products are low shrinkage and low stress restoration. "Crystallinity" as used herein refers to regularity and order within a material resulting in a heat of fusion of at least 1.0 J/g at and below 50°C. Heat of Fusion as used herein refers to enthalpy of fusion as determined by ASTM 793-95. Percent crystallinity is determined by measuring the heat of fusion using differential scanning calorimetry according to ASTM test method E.793-95.

The present invention is further illustrated by the following Example, but this Example should not be construed as limiting the scope of the invention.

An example of a useful adhesive formulation according to the present invention is given in Table I.

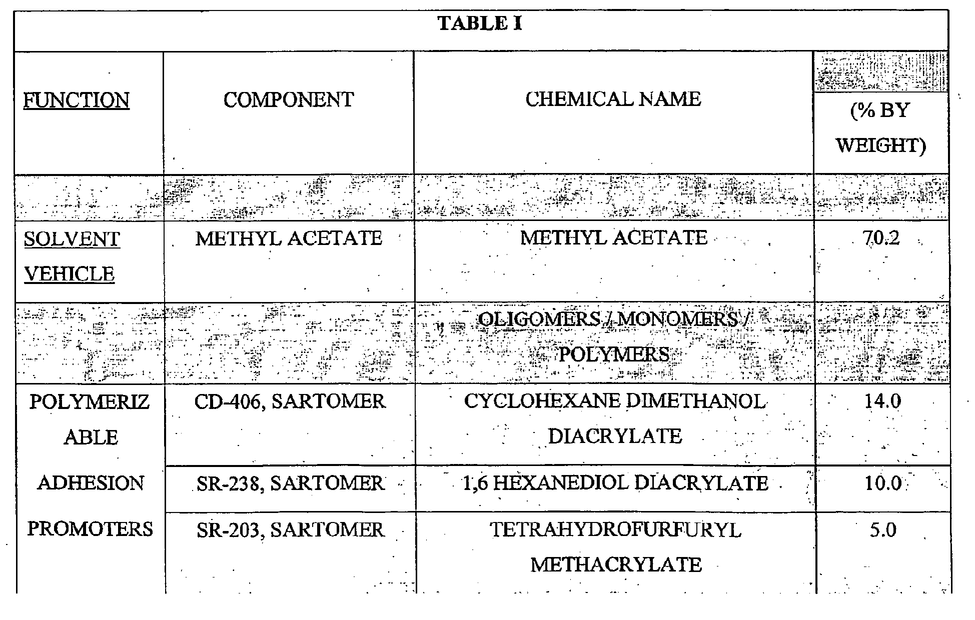

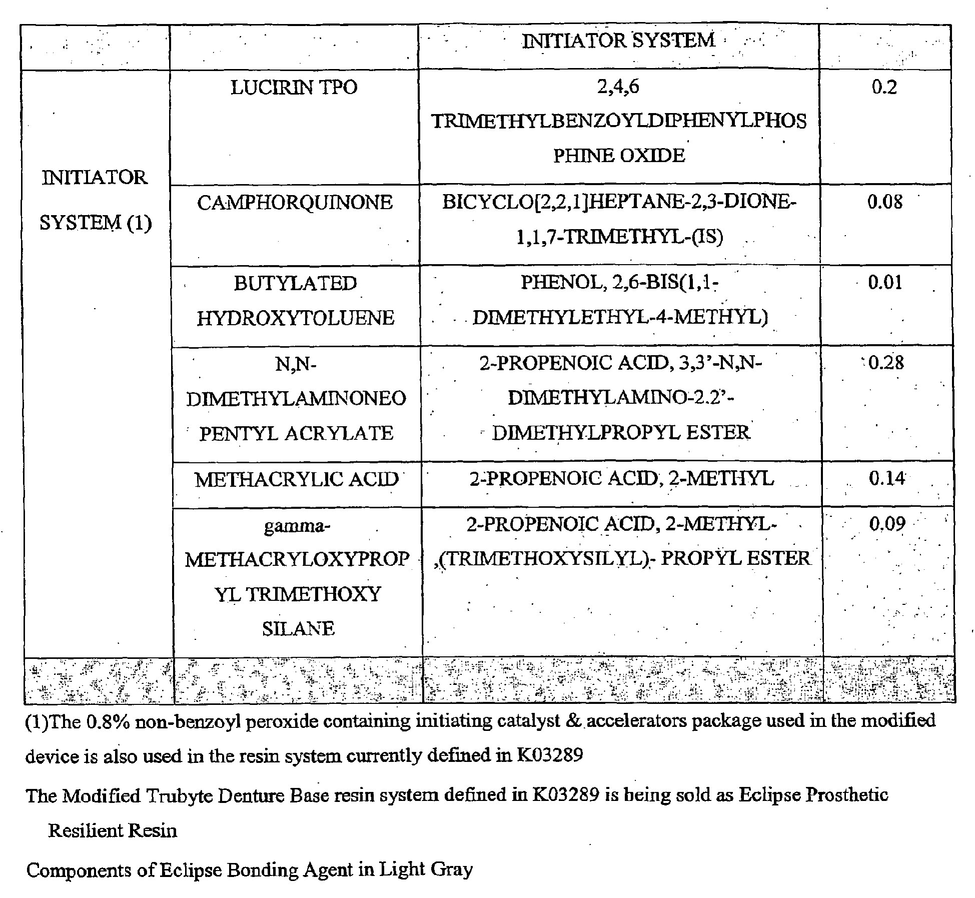

### General Experimental

Tooth bond test specimen design and bond strength performance testing for as used herein are based on the procedures established in International Standard ISO-3336-93 Dentistry - Synthetic Polymer Teeth. Although ISO-3336 has an essentially qualitative definition of what is acceptable tooth bond strength, based on the mode of failure being cohesive or adhesive in nature, this study adopted its' use because of the specific procedures it suggests for specimen geometry, fabrication, and testing. By standardizing tooth geometries, adherend geometries, specimen preparation procedures, and specimen test procedures it has been able herein to quantitatively assess those factors of significance which affect the strength and longevity of the bond between synthetic polymer teeth and acrylic denture base resins. Comparative analysis of bond strength test results both before and after various exposure conditions allow for assessment of both significant differences and substantial equivalence.

### Experimental Procedures

### Specimen Geometry

Tooth bond test specimens as used herein consist of beams of denture base resin approximately 60 mm long by 13 mm deep by 6 mm thick. Prepared (i.e. subjected to the experimental treatment whether it be nothing, bonding agent, mechanical retention, wax then bonding agent etc.) ridgelaps of acrylic denture teeth (Bioform S-mould anterior centrals and laterals) are cured onto the 6 mm edge such that the adhesive interface only includes the ridgelap of the tooth, not the cervical neck or the sub-incisal lingual surface. ISO-3336 Figure 3 (not shown herein) has a general depiction of this geometry. Instead of using all 6 anterior teeth, the canines are excluded and a sample set typically consists of two bars of 2-centrals and two laterals each. Therefore, there are a total of 8 teeth per sample set.

### Fixturing and "Fast-Fracture"Testing

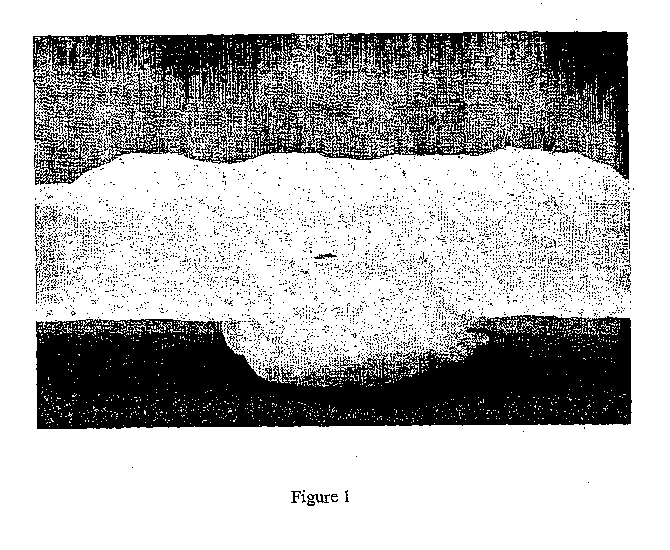

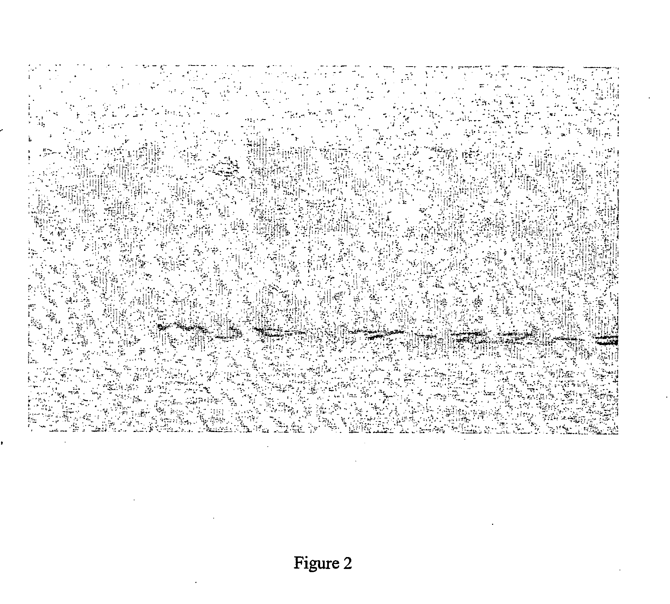

The beams containing the teeth are rigidly clamped in Instron Style 2716 self-tightening wedge action grips. The incisal edges on the lingual aspect are loaded in the labial direction at a displacement rate of 1.25 mm / min. until either cohesive or adhesive failure occurs. The peak force in newton (lbs.-force) is recorded along with the mode of failure. ISO-3336 Figure 3c (not shown) provides a diagram of the fixturing and loading geometry. Typically, cohesive failure occurs at relatively high loads, indicative of a stronger bond, while adhesive failure occurs at relatively low loads, indicative of a poor bond. Figures 1 and 2 below depict fracture surfaces of bond test specimens that are cohesive and adhesive, respectively

### Exposure Conditions

The fast-fracture bond tests were conducted to characterize the bond strengths subsequent to one of three different types of specimen conditioning or exposure scenarios:
1. 24-hours post fabrication which is termed "baseline" condition
2. 24-hours post immersion thermocycling 2125 times between 5°C and 55°C which is Prosthetics' internal assessment of bond "longevity."
3. 24-hours post simulated masticatory fatigue simulation for 1.4 million loading cycles between 0 and 60 N of load in recirculating synthetic saliva held at 37°C.

### Results

### Comparison to Predicate Device

Table II presents the results of bond strength tests in Fas-Por⁺ pour acrylic denture base resin comparing teeth pretreated according to the direction for use with Trubyte Denture Bond (predicate device) with teeth pretreated with the inventive bonding agent. Bond strengths for teeth treated with the inventive are 2-3 times higher than those treated with Trubyte Denture Bond.

**Table II: Comparative Bond Strengths of Baseline Condition Bioform IPN Teeth Treated with Trubyte Denture Bond vs. Eclipse Bonding Agent in Fas-Por⁺ Pour Acrylic**

| BF-IPN Teeth Bonded to Fas Por+ | Predicate Device (Trubyte Denture Bond) | | Inventive Bonding Agent | |
|---|---|---|---|---|
| Samples with Treated Teeth | Bond Strength in N (lbf) | | Bond Strength in N (lbf) | |
| Sample # | Lateral | Central | Lateral | Central |
| 1 | 174 **(39.10)** | 195 **(43.84)** | 446 **(100.20)** | 374 **(84.03)** |
| 2 | 177 **(39.80)** | 206 **(46.31)** | 414 **(92.97)** | 383 **(86.04)** |
| 3 | 149 **(33.45)** | 170 **(38.11)** | 376 **(84.56)** | 264 **(59.38)** |
| 4 | 138 **(31.11)** | 166 **(37.21)** | 446 (100.20) | 385 **(86.50)** |
| 5 | 148 **(33.19)** | 181 **(40.62)** | 446 (100.20) | 446 (100.20) |
| 6 | 152 **(34.11)** | 192 **(43.21)** | * | 404 **(90.9)** |
| 7 | 170 **(38.27)** | 183 **(41.23)** | * | * |
| 8 | 178 **(40.03)** | 178 **(39.99)** | * | * |
| 9 | 183 **(41.03)** | 171 **(38.46)** | | |
| 10 | * | 168 **(37.66)** | | |
| | | | | |
| Average: | 163 (36.68) | 181 (40.66) | 425 (95.63) | 376 (84.51) |

| | | | | |
|---|---|---|---|---|
| Notes: All values in bold font indicate cohesive-type failure. All values in normal font indicate non-fracture of teeth or resin (Those exceeding 1001bf limit being an acceptable result) "*" Indicates sample slipped in grip prior to test completion, beam too thin. | | | | |

Table III presents the results of bond strength tests in Lucitone 199 packable denture base resin comparing teeth pretreated with Trubyte Denture Bond (predicate device) with teeth pretreated with Eclipse Bonding Agent prior to packing and curing the denture base resin. In both instances the teeth were rinsed with 0.5% wax contaminated "boil-out" water prior to application of the bonding agents. Although both bonding agents are effective under these severely contaminated conditions and result in cohesive type failures, the bond strengths for teeth treated with the Eclipse Bonding Agent are approximately 1.5 times higher than those treated with Trubyte Denture Bond.

**Table III: Comparative Bond Strengths of Baseline Condition for Wax Contaminated Bioform IPN Teeth Treated with Trubyte Denture Bond vs. Eclipse Bonding Agent in L-199 Pack Acrylic**

| BF-IPN Teeth Bonded to L-199 | Predicate Device (Trubyte Denture Bond) | | Inventive Bonding Agent | |
|---|---|---|---|---|
| Samples with Treated Teeth | Bond Strength in N (lbf) | | Bond Strength in N (lbf) | |
| Sample # | Lateral | Central | Lateral | Central |
| 1 | 176 **(39.61)** | 172 **(38.71)** | 353 **(79.41)** | 290 **(65.13)** |
| 2 | 165 **(37.19)** | 174 **(39.21)** | 347 **(78.07)** | 292 **(65.69)** |
| 3 | 141 **(31.69)** | 211 **(47.37)** | 271 **(60.94)** | 248 **(55.84)** |
| 4 | 119 **(26.71)** | 223 **(50.11)** | 237 **(53.21)** | 239 **(53.64)** |
| 5 | 178 **(40.03)** | 168 **(37.71)** | | |
| 6 | 192 **(43.11)** | 178 **(39.97)** | | |
| 7 | 229 **(51.44)** | 178 **(40.11)** | | |
| 8 | 175 **(39.27)** | 223 **(50.13)** | | |
| | | | | |
| Average: | 172 (38.63) | 191 (42.92) | 302 (67.91) | 267 (60.08) |

| | | | | |
|---|---|---|---|---|
| Notes: All values in bold font indicate cohesive-type failure. | | | | |

### Efficacy with Other Pour Acrylic Resins

Table IV below provides results for bond strengths in two other pour acrylic resin systems in addition to Fas-Por⁺. Results for use of the inventive material with Fricke Hi-I resin include bond strengths both before and after immersion thermocycling. Improvements in bond strength achieved from treatment with the inventive material range from 39% (percent) for the base line condition to 46% after thermocycling.

Similarly, for Palapress-Vario pour acrylic, improvements in bond strength due to treatment of teeth with the inventive agent are 26% for the baseline condition and 30% after immersion thermocycling. Bond strengths to Fas-Por⁺ increased 32% for the baseline condition, but were basically unchanged after thermocycling.

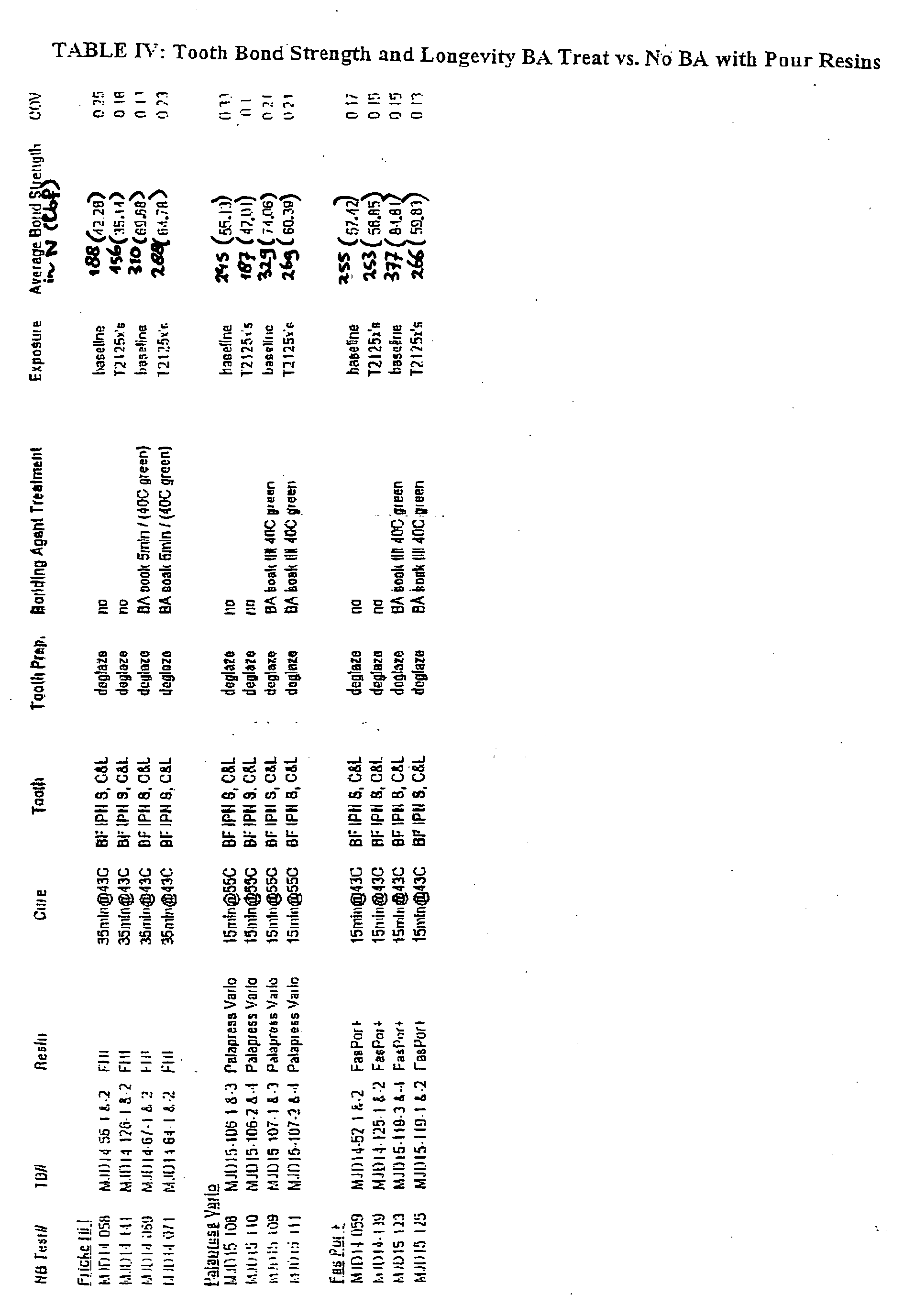

### Efficacy with Eclipse Denture Base Resins

Table V herein presents bond strength summary results for Bioform IPN S-mould teeth set in Eclipse denture base resin. These data compare the results of the currently recommended means of retention for teeth in Eclipse resins, slots and collar grooves, with teeth treated with the bonding agent according to the present invention and no slots and collar grooves. Clearly, the teeth are more effectively retained through the use of the inventive material than with slots and collar grooves under all of the listed conditions.

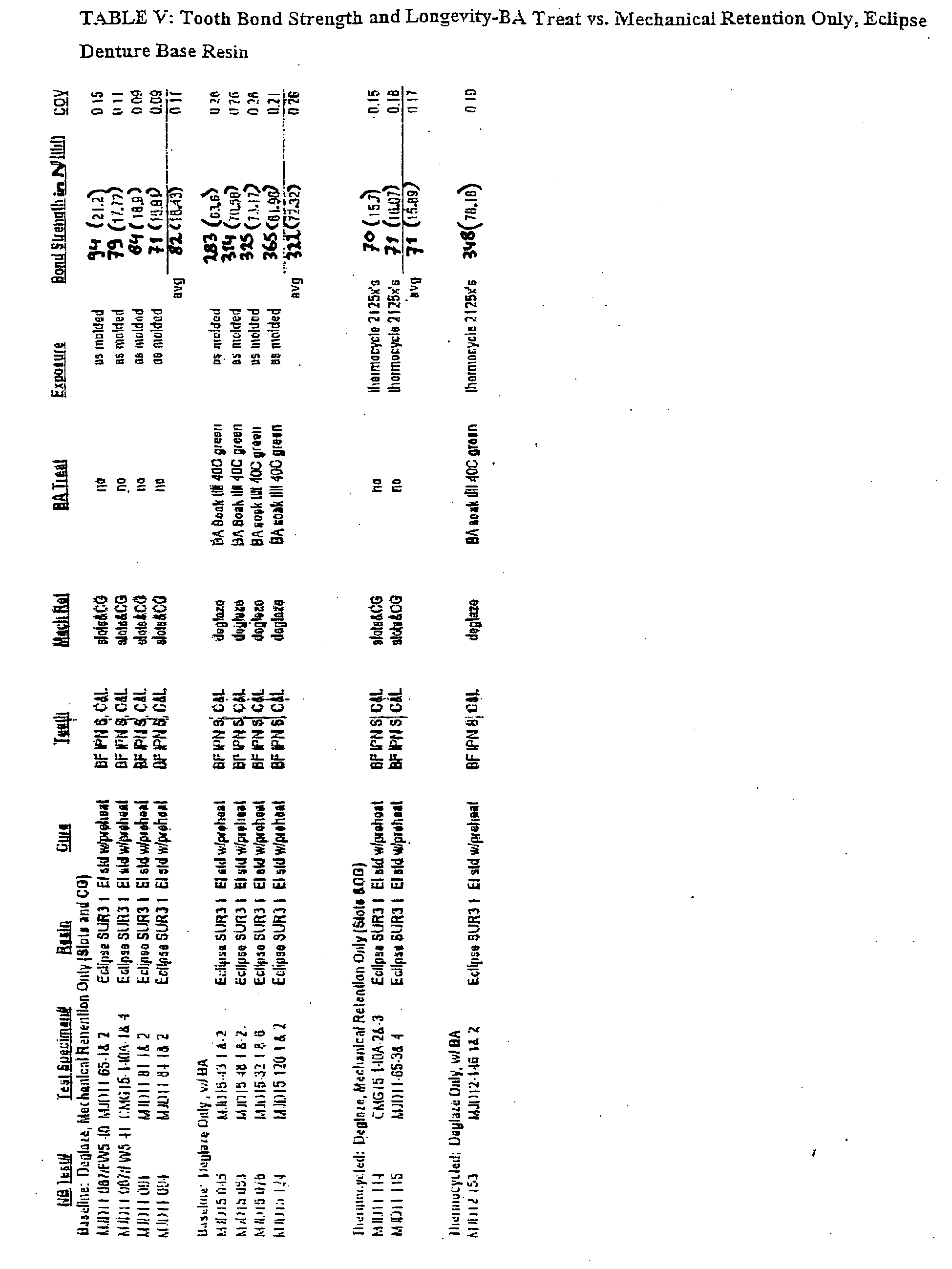

An example of a method according to the invention includes the following steps:
Step1: Prepare Baseplate with ECLIPSE BPR
Step 2: Remove Wax with Clean Dry Wipe
Step 3: Grind Teeth as Necessary
Step 4: To Fit for Occlusion based on Available Vertical Dimension
Step 5: Remove all Residue with an Alcohol-Saturated Wipe
Step 6: Remove Lid From the Circular Tin
Step 7: Add Enough Bonding Agent to Saturate the Foam and Pool to a Depth of 1mm in the Bottom of the Tin
Step 8: Use Tweezers to Place Teeth with Ridgelap Down
Step 9: Fill the Tin with up to 14 Teeth
Step 10: Replace the Lid on the Tin
Step 11: Plug-in the Warming Device to Warm Up
Step 12: Place Tooth-Filled Container on Warming Device For a Period of Five Minutes or Until The 40°C Rectangle on the Temperature Strip Turns Green
Step 13: In a Well-Ventilated Area, Retrieve the Teeth with Tweezers
Step 14: Then Place Teeth on a Clean Paper Towel to Dry For About 1 Minute
Step 15: Set Treated Teeth Back on Baseplate in Eclipse Set-up Resin

It is apparent that a method and composition according to the present invention as described herein provides a contribution to the dental arts. The invention has been described and depicted without attempting to show all of the variations within the scope thereof. The scope of the invention shall be determined by the attached claims.

## Claims

1. A method for bonding an artificial plastic tooth to a denture base comprising the steps of
(a) providing a polymerizable or curable adhesive having
(a1) a solvent,
(a2) an adhesion promoter selected from the group consisting of monomers, dimers, oligomers and polymers, which contains materials having free radically active functional groups having one or more ethylenically unsaturated groups, and
(a3) a cure package for the adhesion promoter;
(b) exposing the plastic tooth to said adhesive in a tin;
(c) placing the tin containing the tooth and the adhesive on a stand-alone electrical warming device and warming the plastic tooth while in contact with the adhesive for softening the artificial plastic tooth and infiltrating the plastic tooth matrix with adhesion promoter chains, and
(c) forming a secure bond between the artificial plastic tooth and the denture base by curing of the adhesive.

2. A method as in claim 1, wherein said solvent is capable of penetrating into the denture base.

3. A method as in claim 2, wherein the solvent is selected from the group consisting of acetone, ethyl acetate, propyl acetate, ethers, methylene chloride, chloroform, cyclohexanone, methyl acetate, methyl ethyl ketone, methyl propyl ketone, tetrahydrofuran and mixtures thereof.

4. A method as in claim 1, wherein said adhesion promoter is selected from the group consiting of mono , di- or polyacrylates and methacrylates such as methyl acrylate, methyl methacrylate, ethyl acrylate, isopropyl methacrylate, n hexyl acrylate, stearyl acrylate, allyl acrylate, glycerol diacrylate, glycerol triacrylate, ethyleneglycol diacrylate, diethyleneglycol diacrylate, triethyleneglycol dimethacrylate, tetraethylene glycol di(meth)acrylate, 1,3-propanediol diacrylate, 1,3-propanediol dimethacrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, pentaerythritol triacrylate, pentaerythritol tetraacrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)plienyl]propane (Bis-GMA); 2,2-bis[4-(acryloyloxy ethoxy)phenyl]propane; 2,2-bis[4-(methacryloyloxy ethoxy)phenyl]propane (or ethoxylated bisphenol-A dimethacrylate) (EBPADMA); urethane di(meth)acrylate (UDMA), diurethane dimethacrylate (DUDMA), 4,13-dioxo 3,14-dioxa-5,12-diazahexadecane-1,16-diol diacrylate, 4,13-dioxo-3,14-dioxa 5,12-diazahexadecane-1,16 -diol dimethacrylate; polyurethane dimethacrylate (PUDMA); alkoxylated pentaerythritol tetraacrylate; polycarbonate dimethacrylate (PCDMA); the bis-acrylates and bis - methacrylates of polyethylene glycols; copolymerizable mixtures of acrylated monomers; acrylated oligomers; acidic monomers such as dipentaerythritol pentacrylate phosphoric acid ester (PENTA); bis[2-(methacryloxyloxy) ethyl]phosphate; and vinyl compounds such as styrene, diallyl phthalate, divinyl succinate, divinyl adipate, divinylphthalate and mixtures thereof.

5. A method as in claim 1 wherein said cure package comprises a peroxide.

6. A method as in claim 5 wherein said peroxide is selected from the group consisting of dibenzoyl peroxide, di-p-chlorobenzoyl peroxide, di-2,4-dichlorobenzoyl peroxide, tertiary butyl peroxybenzoate, methyl ethyl ketone peroxide, ditertiary butyl peroxide, dicumyl peroxide and cumene hydroperoxide.

7. A method as in claim 1 wherein said adhesive further comprises a polymerization accelerator.

8. A method as in claim 7 wherein said polymerization accelerator is a tertiary amine.

9. A method as in claim 8 wherein said tertiary amine is selected from the group consiting of N, N-dimethyl aminoneopentyl acrylate, N, N-dimethyl aminoethyl acrylate, N, N-dimethyl aminoethyl methacrylate, N-methyl diethanolamine; ethyl 4 (dimethylamino)benzoate (EDMAB); 2-[4-(dimethylamino)phenyl] ethanol; N, N-dimethyl-p-toluidine (DMPT); dihydroxyethyl-p-toluidine (DHEPT); bis(hydroxyethyl)-p-toluidine; and triethanolamine.

10. A method as in claim 1 wherein said cure package comprises a photoinitiator.

11. A method as in claim 1 wherein said adhesion promoter is crystalline.

12. A method as in claim 11 wherein said adhesion promoter has a heat of fusion of at least 1.0 J/g at and below 50 °C .

## Patentansprüche

1. Verfahren zum Verbinden eines künstlichen Plastikzahns mit einer Gebissbasis, umfassend die Stufen eines
(a) Bereitstellens eines polymerisierbaren oder härtbaren Kunststoffs mit
(a1) einem Lösungsmittel,
(a2) einem Haftvermittler, ausgewählt aus der Gruppe, bestehend aus Monomeren, Dimeren, Oligomeren und Polymeren, die Materialien enthalten, die freie radikalisch aktive funktionelle Gruppen mit einer oder mehreren ethylenisch ungesättigten Gruppen aufweisen, und
(a3) einen Härter für den Haftvermittler;
(b) Aussetzen des Plastikzahns einem Adhesiv in einem Behälter;
(c) Anordnen des Behälters, der den Zahn und das Adhesiv enthält, in eine eigenständige elektrische Erwärmungseinrichtung und Erwärmen des Plastikzahns zur Erweichung des künstlichen Plastikzahns während dieser in Kontakt steht mit dem Adhesiv und
(d) Bildung einer festen Verbindung zwischen dem künstlichen Plastikzahn und der Gebissbasis durch Aushärten des Adhesivs.

2. Verfahren nach Anspruch 1, wobei das Lösungsmittel geeignet ist zur Penetration der Gebissbasis.

3. Verfahren nach Anspruch 2, wobei das Lösungsmittel ausgewählt wird aus der Gruppe, bestehend aus Aceton, Ethylacetat, Propylacetat, Ether, Methylenchlorid, Chloroform, Cyclohexanon, Methylacetat, Methylethylketon, Methylpropylketon, Tetrahydrofuran und Gemischen davon.

4. Das Verfahren nach Anspruch 1, wobei der Haftvermittler ausgewählt wird aus der Gruppe, bestehend aus Mono-, Di- oder Polyacrylaten und Methacrylaten, wie Methylacrylat, Methylmethacrylat, Ethylacrylat, Isopropylmethacrylat, n-Hexylacrylat, Stearylacrylat, Allylacrylat, Glycerindiacrylat, Glycerintriacrylat, Ethyleneglycoldiacrylat,
Diethylenglycoldiacrylat, Triethylenglycoldimethacrylat, Tetraethylenglycoldi(meth)acrylat, 1,3-Propanedioldiacrylat, 1,3-Propanedioldimethacrylat, Trimethylolpropantri(meth)acrylat, 1,2,4-Butanetrioltrimethacrylat, 1,4-Cyclohexandioldiacrylat, 1,4-Cyclohexandioldimethacrylat, 1,6-Hexandioldi(meth)acrylat, Pentaerythritoltriacrylat, Pentaerythritoltetraacrylat, Pentaerythritoltetramethacrylat, Sorbitolhexacrylat, 2,2-bis[4-(2-hydroxy-3-acryloyloxypropoxy)phenyl]propan; 2,2-bis[4-(2-hydroxy-3-methacryloyloxypropoxy)phenyl]propan (Bis-GMA); 2,2-bis[4-(acryloyloxyethoxy)phenyl]propan; 2,2-bis[4-(methacryloyloxyethoxy)phenyl]propan (oder ethoxylierte bisplienol-A-dimethacrylat) (EBPADMA); Urethandi(meth)acrylat (UDMA), Diurethandimethacrylat (DUDMA), 4,13-dioxo 3,14-dioxa-5,12-diazahexadekan-1,16-dioldiacrylat, 4,13-dioxo-3,14 dioxa-5,12-diazahexadekan-1,16-dioldimethacrylat; Polyurethandimethacrylat (PUDMA); alkoxyliertes Pentaerythritoltetraacrylat; Polycarbonatdimethacrylat (PCDMA); die bis-Acrylate und bis-Methacrylate von Polyethylenglycolen; copolymerisierte Gemische aus acrylierten Monomeren; acrylierte Oligomere; saure Monomere wie Dipentaerythritolpentacrylatphosphorsäureester (PENTA); bis[2-(methacryloxyloxy)-ethyl]phosphat; und Vinylverbindungen wie Styrol, Diallylphthalat, Divinylsuccinat, Divinyladipat, Divinylphthalat und Gemische davon.

5. Das Verfahren nach Anspruch 1, wobei der Härter ein Peroxid umfasst.

6. Das Verfahren nach Anspruch 5, wobei das Peroxid ausgewählt wird aus der Gruppe, bestehend aus Dibenzoylperoxid, Di-p-chlorbenzoylperoxid, Di-2,4-dichlorbenzoylperoxid, Tertiabutylperoxybenzoat, Methylethylketonperoxid, Diteriabutylperoxid, Dicumylperoxid und Cumolhydroperoxid.

7. Das Verfahren nach Anspruch 1, wobei das Adhesiv ferner einen Polymerisationsbeschleuniger enthält.

8. Das Verfahren nach Anspruch 7, wobei der Polymerisationsbeschleuniger ein tertieres Amin ist.

9. Verfahren nach Anspruch 8, wobei das tertiere Amin ausgewählt ist aus der Gruppe, bestehend aus N,N-Dimethylaminoneopentylacrylat, N,N-Dimethylaminoethylacrylat, N,N-Dimethylaminoethylmethacrylat, N-Methyldiethanolamin; Ethyl-4-(dimethylamino)benzoat (EDMAB); 2-[4-(Dimethylamino)phenyl]ethanol; N,N-Dimethyl-p-toluidin (DMPT); Dihydroxyethyl-p-toluidin (DHEPT); bis(hydroxyethyl)-p-toluidin; sowie Triethanolamin.

10. Das Verfahren nach Anspruch 1, wobei der Härter einen Photoinitiator umfasst.

11. Das Verfahren nach Anspruch 1, wobei der Adhäsionsvermittler kristallin ist.

12. Verfahren nach Anspruch 11, wobei der Adhäsionsvermittler eine Schmelzwärme von mindestens 1,0 J/g aufweist bei und unter 50°C.

## Revendications

1. Procédé pour lier une dent artificielle en matière plastique à une base de prothèse dentaire, comprenant les étapes consistant
(a) à fournir un adhésif polymérisable ou durcissable comprenant
(a1) un solvant,
(a2) un activateur d'adhérence choisi dans le groupe consistant en des monomères, dimères, oligomères et polymères, qui contient des matières ayant des groupes fonctionnels à activité de radicaux libres comprenant un ou plusieurs groupes à insaturation éthylénique, et
(a3) une formulation de durcisseur pour l'activateur d'adhérence ;
(b) à exposer la dent en matière plastique audit adhésif dans une boîte ;
(c) à placer la boîte contenant la dent et l'adhésif sur un dispositif de chauffage électrique autonome et à chauffer la dent en matière plastique tandis qu'elle est en contact avec l'adhésif pour le ramollissement de la dent artificielle en matière plastique et l'infiltration des chaînes de l'activateur d'adhérence dans la matrice de dent en matière plastique, et
(d) à former une liaison résistante entre la dent artificielle en matière plastique et la base de prothèse dentaire par durcissement de l'adhésif.

2. Procédé suivant la revendication 1, dans lequel ledit solvant est capable de pénétrer dans la base de prothèse dentaire.

3. Procédé suivant la revendication 2, dans lequel le solvant est choisi dans le groupe consistant en l'acétone, l'acétate d'éthyle, l'acétate de propyle, des éthers, le chlorure de méthylène, le chloroforme, la cyclohexanone, l'acétate de méthyle, la méthyléthylcétone, la méthylpropylcétone, le tétrahydrofuranne et leurs mélanges.

4. Procédé suivant la revendication 1, dans lequel ledit activateur d'adhérence est choisi dans le groupe consistant en des mono-, di- ou polyacrylates et méthacrylates tels que l'acrylate de méthyle, le méthacrylate de méthyle, l'acrylate d'éthyle, le méthacrylate d'isopropyle, l'acrylate de n-hexyle, l'acrylate de stéaryle, l'acrylate d'allyle, le diacrylate de glycérol, le triacrylate de glycérol, le diacrylate d'éthylèneglycol, le diacrylate de diéthylèneglycol, le diméthacrylate de triéthylèneglycol, le di(méth)acrylate de tétraéthylèneglycol, le diacrylate de 1,3-propanediol, le diméthacrylate de 1,3-propanediol, le tri(méth)acrylate de triméthylolpropane, le triméthacrylate de 1,2,4-butanetriol, le diacrylate de 1,4-cyclohexanediol, le diméthacrylate de 1,4-cyclohexanediol, le di(méth)acrylate de 1,6-hexanediol, le triacrylate de pentaérythritol, le tétraacrylate de pentaérythritol, le tétraméthacrylate de pentaérythritol, l'hexacrylate de sorbitol, le 2,2-bis[4-(2-hydroxy-3-acryloyloxypropoxy)phényl]propane ; le 2,2-bis [4- (2-hydroxy-3-méthacryloyloxy-propoxyphényl] propane (Bis-GMA) ; le 2,2-bis[4-(acryl-oyloxyéthoxy)phényl]propane ; le 2,2-bis[4-(méthacryloyl-oxyéthoxy)phényl]propane (ou diméthacrylate de bisphénol-A éthoxylé) (EBPADMA) ; le di(méth)acrylate d'uréthane (UDMA); le diméthacrylate de diuréthane (DUDMA) ; le diacrylate de 4,13-dioxo-3,14-dioxa-5,12-diazahexadécane-1,16-diol, le diméthacrylate de 4,13-dioxo-3,14-dioxa-5,12-diaza-hexadécane-1,16-diol, le diméthacrylate de polyuréthanne (PUDMA) ; un tétraacrylate de pentaérythritol alkoxylé ; le diméthacrylate de polycarbonate (PCDMA) ; les bis-acrylates et bis-méthacrylates de polyéthylèneglycols ; des mélanges copolymérisables de monomères acrylés ; des oligomères acrylés ; des monomères acides tels que l'ester d'acide
phosphorique de pentaacrylate de dipentaérythritol (PENTA); le phosphate de bis[2-(méthacryloxyloxy)éthyle] ; et des composés vinyliques tels que le styrène, le phtalate de diallyle, le succinate de divinyle, l'adipate de divinyle, le phtalate de divinyle et leurs mélanges.

5. Procédé suivant la revendication 1, dans lequel ladite formulation de durcisseur comprend un peroxyde.

6. Procédé suivant la revendication 5, dans lequel ledit peroxyde est choisi dans le groupe consistant en le peroxyde de dibenzoyle, le peroxyde de di-p-chlorobenzoyle, le peroxyde de di-2,4-dichlorobenzoyle, le peroxybenzoate de tertiobutyle, le peroxyde de méthyléthylcétone, le peroxyde de di-tertiobutyle, le peroxyde de dicumyle et l'hydroperoxyde de cumène.

7. Procédé suivant la revendication 1, dans lequel ledit adhésif comprend en outre un accélérateur de polymérisation.

8. Procédé suivant la revendication 7, dans lequel ledit accélérateur de polymérisation est une amine tertiaire.

9. Procédé suivant la revendication 8, dans lequel ladite amine tertiaire est choisie dans le groupe consistant en l'acrylate de N,N-diméthylaminonéopentyle, l'acrylate de N,N-diméthylaminoéthyle, le méthacrylate de N,N-diméthylaminoéthyle, la N-méthyldiéthanolamine ; le 4(diméthylamino)benzoate d'éthyle (EDMAB) ; le 2-[4-(diméthylamino)phényl]éthanol ; la N,N-diméthyl-p-toluidine (DMPT) ; la dihydroxyéthyl-p-toluidine (DHEPT) ; la bis-(hydroxyéthyl)-p-toluidine et la triéthanolamine.

10. Procédé suivant la revendication 1, dans lequel ladite formulation de durcisseur comprend un photo-initiateur.

11. Procédé suivant la revendication 1, dans lequel ledit activateur d'adhérence est cristallin.

12. Procédé suivant la revendication 11, dans lequel ledit activateur d'adhérence a une chaleur de fusion d'au moins 1,0 J/g à une température égale ou inférieure à 50°C.
